Europäisches Patentamt

European Patent Office

Office européen des brevets

(11) Veröffentlichungsnummer: **0 129 503**
**A2**

(12) **EUROPÄISCHE PATENTANMELDUNG**

(21) Anmeldenummer: **84730068.8**

(22) Anmeldetag: **18.06.84**

(51) Int. Cl.³: **A 61 N 1/36**

(30) Priorität: **18.06.83 DE 3322284**

(43) Veröffentlichungstag der Anmeldung:
**27.12.84 Patentblatt 84/52**

(84) Benannte Vertragsstaaten:
**AT BE CH DE FR GB IT LI LU NL SE**

(71) Anmelder: **BIOTRONIK Mess- und Therapiegeräte GmbH & Co Ingenieurbüro Berlin Sieversufer 8 D-1000 Berlin 47(DE)**

(72) Erfinder: **von Leitner, Enz-Rüdiger, Dr. Kiefernweg 8 D-1000 Berlin 19(DE)**

(72) Erfinder: **Rexhausen, Hermann Mellinger Strasse 48 D-3200 Hildesheim(DE)**

(74) Vertreter: **Christiansen, Henning, Dipl.-Ing. Unter den Eichen 108a D-1000 Berlin 45(DE)**

(54) **Herzschrittmacher.**

㊼ Herzschrittmacher mit Mitteln zur Erzeugung von besonderen Stimulationsimpulsen zur Tachycardie-Terminierung, wobei Schaltmittel vorgesehen sind, welche die Impulse zur Tachycardie-Terminierung in ihrer Intensität derart einstellen, daß sie die Stimulationsschwelle bei regulärer Stimulation nicht erreichen.

EP 0 129 503 A2

Croydon Printing Company Ltd.

BIOTRONIK Meß- und Therapiegeräte     18. Juni 1984
GmbH & Co Ingenieurbüro Berlin
D-1000 Berlin

B83.8-EU

---

### Herzschrittmacher

---

B e s c h r e i b u n g

Die Erfindung betrifft einen Herzschrittmacher mit Mitteln
zur Tachycardie-Terminierung der im Oberbegriff des Anspruchs 1 angegebenen Art.

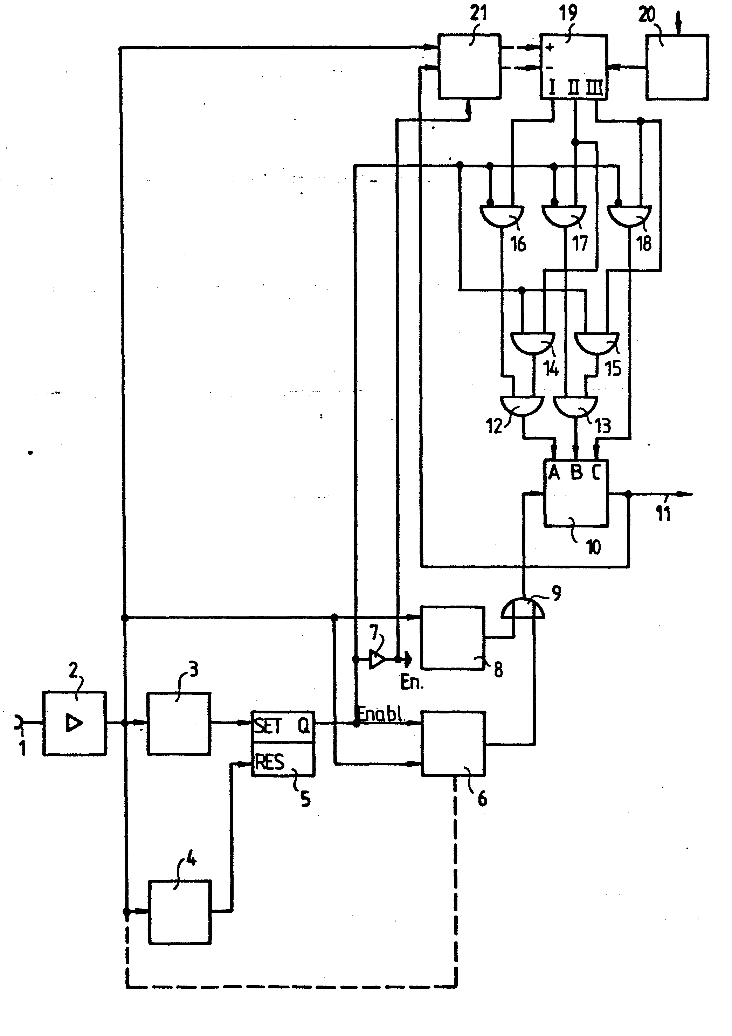

Derartige Schrittmacher sind in verschiedenen Ausführungen
bekannt, wobei unterschiedliche Therapieformen bekannt sind. Eine Übersicht über die bisher gebräuchlichen Formen der Schrittmachertherapie findet sich beispielsweise in Lüderitz, Berndt: "Elektrische Stimulation des Herzens", 1979, 6.2.2: "Schrittmachertherapie". Bei den bekannten Möglichkeiten zur Terminierung von vorzugsweise supraventrikulären und ventrikulären Tachycardien mittels antitachykarder Stimulationstherapie werden nach dem Erkennen des Vorliegens einer Tachycardie zusätzliche Stimulationsimpulse innerhalb des Re-Entry-Zyklus abgegeben, welche derart zeitlich gesteuert sein müssen, daß sie entsprechend der zugehörigen Modellvorstellung (aaO., Seite 15, Abb. 1.8) in eine erregbare Lücke fallen, so daß durch Depolarisation des Myokardbereichs in der Umgebung der Schrittmacherelektrode die Erregungsfront ein Gebiet vorfindet, welches bereits refraktär ist und die kreisende Erregung unterbrochen wird.

Nach den bisherigen Vorstellungen auf dem Gebiete der Elektrophysiologie des Herzens war eine derartige Terminierung unter Depolarisation des betreffenden Gewebebereichs nur dann möglich, wenn die Stimulation mit einer Intensität ausgeführt wurde, welche die jeweilige Stimulationsschwelle überschreitet. Dabei wird unter einem derartigen Impuls ein Stimulationsereignis verstanden, welches bezüglich seiner Intensität - entsprechend den vorliegenden individuellen Gegebenheiten - aufgrund seiner Amplitude, Impulsenergie, des zeitlichen Anstiegs etc. in der Lage ist, einen QRS-Komplex auszulösen. Die Stimulationsschwelle bildet dabei denjenigen Grenzwert, der bei der regulären Schrittmachertherapie mittels Elektrostimulation des Herzens von den einzelnen Stimulationsimpulsen übertroffen sein muß, damit diese wirksam werden. Die betreffende Schwelle wird bei der Anwendung von Schrittmachern jeweils für jeden Patienten individuell ermittelt und eingestellt. Die Stimulationsschwelle ist zudem zeitabhängigen Veränderungen unterworfen.

Die Tachycardie-Terminierung mittels zusätzlicher Stimulationsimpulse bringt nun die Gefahr mit sich, daß diese Impulse in die sogenannte "vulnerable" Phase fallen und damit Kammerflimmern hervorrufen können.

Der im kennzeichnenden Teil des Anspruchs 1 angegebenen Erfindung liegt die Aufgabe zugrunde, einen Schrittmacher der eingangs genannten Gattung anzugeben, der eine gefahrlose Tachycardie-Terminierung ermöglicht.

Besonders vorteilhaft ist dabei, daß bei herabgesetzter Impulsamplitude bzw. -energie, die in einem implantierten Schrittmacher vorhandenen Energiereserven weniger beansprucht werden und damit die erreichbare Implantationszeit vergrößert ist.

Die Erfindung beruht auf der überraschenden Erkenntnis, daß bei der Tachycardie-Terminierung bereits durch solche Impulse eine Depolarisation des Myocardgewebes mit darauffolgender Ausbildung eines refraktären Zustandes mit Unterbrechung der weiteren Ausbreitung der Erregungsfront erreicht werden kann, welche zur regulären Stimulation nicht ausreichen, wobei das Hervorrufen einer Kontraktion bzw. des unerwünschten Ergebnisses einer Stimulation in die vulnerable Phase mit Sicherheit verhindert ist.

/4

0129503

Bei bevorzugten Weiterbildungen der Erfindung wird die Anpassung des Stimulationsimpulses an die Stimulationsschwelle durch Amplitudenänderung vorgenommen, wobei Einstellmittel vorgesehen sind, welche die Amplitudenanpassung der Stimulationsamplitude selbsttätig vornehmen und die Intensität der antitachycarden Impulse jeweils um einen vorgegebenen Betrag oder Faktor unterhalb der Stimulationsintensität bei regulärem Betrieb halten. Bei sogenannten multiprogrammierbaren Schrittmachern mit in Stufen einstellbarer Ausgangsamplitude sind zweckmäßigerweise Mittel vorgesehen, welche beim Übergang von der regulären Stimulation zur Tachycardie-Terminierung die Ausgangsamplitude selbsttätig um eine oder mehrere Stufen gegenüber der programmierten Amplitude herabsetzen.

Eine bevorzugte Anwendung der Erfindung ergibt sich mit dem Prinzip der Tachycardie-Terminierung unter Aussendung von Burst-Impulsen, da in diesem Fall eine besonders große Wahrscheinlichkeit besteht, daß ein Zeitfenster erreicht wird, in dem eine Tachycardie-Beendigung möglich ist. Es bedarf dabei keiner aufwendigen Schaltungsmittel, welche eine relative zeitliche Zuordnung der anti-tachycarden Stimulationsimpulse zu anderen Ereignissen im Herzen festlegen, da die Anwendung der erfindungsgemäßen Impulssteuerung gefahrlos ist.

Gemäß der Erfindung wird durch diese Impulse, welche für sich allein nicht zur Auslösung einer Kammerkontraktion ausreichend sind, eine Depolarisation des entsprechenden Myocardbereichs erreicht, wobei ein Refraktärzustand bzw. eine zeitliche Verlängerung des vorhandenen Refraktär-

0129503

zustands erreicht wird, ohne daß die übrigen mit einem die Stimulationsschwelle übertreffenden Impuls verbundenen schädlichen Wirkungen eintreten.

Die Auslösung der Impulse zur Tachycardie-Terminierung kann entweder selbsttätig oder manuell gesteuert erfolgen. Im Falle der automatischen Auslösung derselben wird eines der bekannten Kriterien zur Erkennung von Tachycardien herangezogen, wie es entsprechend bei den bekannten Systemen verwendet wird. Ein bevorzugtes Auslösungskriterium bildet dabei die Änderung der Zeitdifferenz zwischen aufeinanderfolgenden natürlichen Ereignissen, d.h. es wird ein Frequenzsprung der im Herzen aufgenommenen Signale ausgewertet, der einen vorgegebenen Schwellwert überschreitet. Das Ende der Tachycardie wird angenommen, wenn sich wieder die normale Herzfrequenz eingestellt hat.

Bei einer anderen vorteilhaften Variante der Erfindung wird die Stimulationsamplitude statt von außen gesteuert durch Programmierung selbsttätig in Abhängigkeit vom Stimulationserfolg variiert. Dabei wird die Amplitude heraufgesetzt, wenn auf einen Stimulationsimpuls hin keine Herzaktion ausgelöst wurde und in vorgegebenen (größeren) Zeitabständen jeweils um eine Stufe herabgesetzt, wenn eine Anzahl von aufeinanderfolgenden Stimulationen erfolgreich waren. Die Festlegung der Amplitude der Tachycardie-Terminierungs-Impulse folgt jeweils auf einem entsprechend geringeren Niveau.

In der einzigen Figur ist das Blockschaltbild eines Herzschrittmachers wiedergegeben, der die erfindungsgemäßen Mittel zur Tachycardie-Terminierung aufweist.

/6

Von einem Elektrodenanschluß 1 gelangen die im Herzen aufgenommenen Signale zu einem Eingangsverstärker 2, welcher nach der entsprechenden Ausfilterung von Störsignalen etc. ein Ausgangssignal abgibt, das Impulse aufweist, die entsprechende Herzereignisse repräsentieren.

Die Ausgangssignale des Verstärkers 2 werden zwei Schaltungen 3 bzw. 4 zugeführt, welche in der Lage sind, den Beginn (Block 3) und das Ende (Block 4) einer Tachycardie zu erkennen. Diese Schaltungen sind prinzipiell bekannt und werten die Zeitfolge der im Herzen aufgenommenen Signale natürlichen Ursprungs aus, wobei das Überschreiten- bzw. Unterschreiten einer vorgegebenen Impulsrate die Abgabe eines Steuerimpulses bewirkt, welcher dem Setz- bzw. Rücksetz-Eingang eines nachfolgenden Flip-Flops 5 zugeführt wird.

Wenn das Flip-Flop 5 beim Vorhandensein von Tachycardien gesetzt ist, wird über einen entsprechenden Eingang ein nachgeschalteter Zeitgeber für die Impulse zur Tachycardie-Terminierung aktiviert. Während, wenn das Flip-Flop 5 nicht gesetzt ist, sein Q-Ausgang sich also im Zustand "L" befindet, über einen Inverter 7 ein Zeitgeber 8 aktiviert wird, welcher die übliche Abgabe von Stimulationsimpulsen - beispielsweise nach dem die Demand-Prinzip - veranlaßt. Die Steuerimpulse der Stufen 6 und 8 werden über ein ODER-Gatter 9 zusammengefaßt und veranlassen eine Ausgangsstufe 10 zur Abgabe von Stimulationsimpulsen an einen Stimulationsausgang 11, der mit der im Herzen verankerten Elektrode verbunden ist. Die Ausgangsstufe 10 kann über zusätzliche Eingänge A bis C zur Abgabe von Impulsen unterschiedlicher Amplitude veranlaßt werden, deren Zeitpunkt jedoch in jedem Fall durch das Ausgangssignal des ODER-Gatters 9 bestimmt wird.

Das Tachycardie-Terminierungs-Steuerstufe 6 gibt bei Aktivierung in fortgesetzter Folge Impulssalven ab, deren zeitliche Lage von den Herz- oder Stimulationsereignissen abhängig sein kann, wie es bereits aus älteren Veröffentlichungen bekannt ist. (Die entsprechende Signalübertragung ist durch eine Verbindung vom Ausgang des Verstärkers 2 zum Block 6 angedeutet.)

Die Einstellung der Ausgangsamplitude mittels der Ausgangsstufe 10 erfolgt über die Eingänge A, B oder C, wobei die Ansteuerung eines dieser Eingänge jeweils eine bestimmte Amplitude der Ausgangssignale bewirkt und die Ansteuerung des Eingangs C die Einstellung der Maximal-Amplitude veranlaßt. (Die Anzahl der einstellbaren Stufen ist lediglich beispielhaft gewählt und kann entsprechend den gestellten Anforderungen verändert werden.)

Die Eingänge A und B werden über ODER-Gatter 12 und 13 angesteuert, deren einem Eingang jeweils die Ausgangssignale von UND-Gattern 14 und 15 zugeführt werden und zu deren anderem Eingang die Ausgangssignale von UND-Gattern 16 bzw. 17 gelangen. Das Ausgangssignal eines UND-Gatters 18 wird direkt dem Eingang C der Ausgangsstufe 10 zugeführt. Jeweils ein Eingang der UND-Gatter 16, 17 bzw. 18 ist mit einem der Ausgänge I, II bzw. III eines Programmspeichers 19 verbunden, wobei die Aktivierung eines der Ausgänge I, II oder III ebenfalls einer jeweils einge-

/8

stellten Stimulationsamplitude entspricht. Wenn der Q-Ausgang des Flip-Flops 5 nicht aktiviert ist, sind die UND-Gatter 16, 17 und 18 durchgeschaltet, da das Signal des Q-Ausgangs des Flip-Flops 5 den invertierenden Eingängen dieser UND-Gatter zugeleitet wird. Bei Normalbetrieb des Herzschrittmachers wird damit bei aktiviertem Ausgang I des Programmspeichers 9 der Eingang A der Ausgangsstufe 10 angesteuert, bei Aktivierung des Ausgangs II der Eingang B und so weiter.

Ist jedoch der Ausgang Q des Flip-Flops 5 im "H"-Zustand, so werden statt der UND-Gatter 16 bis 18 die UND-Gatter 14 und 15 aktiviert, so daß bei einem Ausgangssignal am Ausgang II des Programmspeichers 19 der Eingang A der Ausgangsstufe und bei Aktivierung des Ausgangs III der Eingang B der Amplitudenausgangsstufe 10 angesteuert werden. Die Amplitude der Stimulationsimpulse ist daher im Falle der Tachycardie-Terminierung jeweils um eine Stufe kleiner als bei der regulären Stimulation.

Der Programmspeicher 19 wird durch ein Programmierteil 20 angesteuert, welches lediglich als Block widergegeben ist, da derartige Schaltungen zum Stand der Technik gehören.

Des weiteren ist ein Block 21 gestrichelt widergegeben, welcher ebenfalls auf den Programmspeicherteil 19 einwirkt, in dem die momentane Stimulationsamplitude festgehalten ist. Der Block 21 bildet eine Effektivitätskontrolle, wobei der entsprechenden Verarbeitungsstufe die Signale am Ausgang des Eingangsverstärkers 2 und am Ausgang der Ausgangsstufe 10 zugeführt werden. Wird innerhalb einer vorgegebenen Zeitdauer nach Abgabe eines Stimulationsimpulses am Ausgang 10 kein Signal am Ausgang des Eingangsverstärkers 2 aufgenommen, so erfolgt eine Heraufsetzung der Stimulationsimpulse um eine Stufe. (+-Eingang
der Stufe 19). Statt des Ausgangs I würde also der Ausgang II des Programmspeichers aktiviert werden oder aber
eine Heraufsetzung der Ausgangsamplitude auf eine entsprechend höhere Intensitätsstufe erfolgen.

Wird dagegen innerhalb des vorgegebenen Zeitraums von
einigen Millisekunden eine reguläre Herzantwort festgestellt, so wird nach einer vorgegebenen Anzahl von regulären Stimulationen die Stimulationsimpulsamplitude über
den Eingang "-" des Programmspeichers 19 um eine Stufe
herabgesetzt. Diese Funktionen der Stufe 21 für die Effektivitätskontrolle finden lediglich während der normalen
Stimulation statt, da die Stufe 21 ebenfalls vom Ausgang
des Inverters 7 über einen zusätzlichen Eingang aktiviert
wird. Auf diese Weise wird erreicht, daß die Stimulationsamplitude der tatsächlichen aktuellen Stimulationsschwelle
des Patienten nachgeführt wird und im Falle der Umschaltung auf den Betriebszustand der Tachycardie-Terminierung
eine Stimulationsamplitude gewählt wird, welche unterhalb
dieser aktuellen Stimulationsschwelle liegt.

Die Stimulationsdaten für die erfolgreiche Terminierung
von Tachycardien mit Raten von 120 bis 240 ($1/_{min}$) betrugen:

Bursts von 10 bis 30 Impulsen mit einer Rate von
800 ($1/_{min}$)

Impulsbreite 2 ms

Impulsstrom 10 bis 20 % unterhalb der Stimulationsschwelle, d.h. 0,8 bis 1,3 mA.

Die Anwendung der Erfindung ist entsprechend bei anderen
Verfahren zur Tachycardie-Terminierung, wie Doppel-Impulsstimulation, "Scanning" etc. möglich und nicht auf die
beispielhaft beschriebene Ausführung beschränkt.

* * * * *

## <u>P a t e n t a n s p r ü c h e</u>

1.    Herzschrittmacher mit Mitteln zur Erzeugung von besonderen Stimulationsimpulsen zur Tachycardie-Terminierung,

d a d u r c h   g e k e n n z e i c h n e t ,

daß Schaltmittel vorgesehen sind, welche die Impulse zur Tachycardie-Terminierung in ihrer Intensität derart einstellen, daß sie die Stimulationsschwelle bei regulärer Stimulation nicht erreichen.

2.    Herzschrittmacher nach Anspruch 1, d a d u r c h g e k e n n z e i c h n e t , daß die Stimulationsimpulse eine derartige Intensität aufweisen, daß sie den den Fixationsort der Elektrode umgebenden Bereich des Myokards ausreichend gegen das Fortschreiten der Erregungsfront refraktär machen, ohne eine Kammerkontraktion auszulösen.

3.    Herzschrittmacher nach einem der vorangehenden Ansprüche,   d a d u r c h   g e k e n n z e i c h n e t , daß die Schaltmittel die Intensität der Stimulationsimpulse im Vergleich zur regulären Stimulation für einen vorgegeben Zeitraum oder eine vorgegebene Anzahl von Impulsen herabsetzen.

4.    Herzschrittmacher nach Anspruch 3, d a d u r c h
g e k e n n z e i c h n e t ,   daß den Schaltmitteln
Ausgangssignale von Tachycardie-Erkennungsmitteln zugeführt werden, welche im Falle des Erkennens einer Tachycardie die Schaltmittel aktivieren.

5.    Herzschrittmacher nach Anspruch 3, d a d u r c h
g e k e n n z e i c h n e t ,   daß die Schaltmittel durch
manuelle externe Betätigung aktivierbar sind.

6.    Herzschrittmacher nach einem der vorangehenden Ansprüche mit Progammiermitteln zur Einstellung der Intensität der regulären Stimulationsimpulse, d a d u r c h
g e k e n n z e i c h n e t ,   daß für die Tachycardie-
Terminierung die Intensität um eine oder mehrere Stufen
gegenüber der programmierten Intensitätsstufe herabgesetzt
wird.

7.    Herzschrittmacher nach einem der Ansprüche 1 bis 5,
d a d u r c h   g e k e n n z e i c h n e t ,   daß die
Einstellung der Intensität der regulären Stimulationsimpulse durch Mittel zur Effektivitätserkennung bei stufenweiser Einstellung jeweils derart erfolgt, daß bei der
eingestellten Schaltstufe noch ein Stimulationserfolg eintritt, während für die Tachycardie-Terminierung die Intensität um eine oder mehrere Stufen gegenüber der eingestellten Schaltstufe herabgesetzt wird.

/13

8.     Herzschrittmacher nach einem der vorangehenden Ansprüche,    d a d u r c h   g e k e n n z e i c h n e t ,
daß die Schaltmittel im Falle der Tachycardie-Terminierung
eine Herabsetzung der Strom- und/oder Spannungsamplitude
der Stimulationsimpulse bewirken.


9.     Herzschrittmacher nach einem der vorangehenden Ansprüche,    d a d u r c h   g e k e n n z e i c h n e t ,
daß die Impulse zur Tachycardie-Terminierung in Form von
Salven abgegeben werden.

*  *  *  *  *